Europäisches Patentamt

European Patent Office

Office européen des brevets

(11) Publication number: **0 615 723 A1**

# EUROPEAN PATENT APPLICATION

(21) Application number: 93301651.1

(22) Date of filing: **04.03.93**

(51) Int. Cl.5: **A61B 5/026**, A61B 5/0275, G01F 1/712

(43) Date of publication of application:
**21.09.94 Bulletin 94/38**

(84) Designated Contracting States:
**DE GB**

(71) Applicant: **HAMAMATSU PHOTONICS K.K.**
**1126-1 Ichino-cho**
**Hamamatsu-shi**
**Shizuoka-ken (JP)**

(72) Inventor: **Delpy, David Thomas, c/o University**
**College London**
**Shropshire House,**
**11-20 Capper Street**
**London WC1E 6JA (GB)**
Inventor: **Cope, Mark, c/o University College**
**London**
**Shropshire House,**
**11-20 Capper Street**
**London WC1E 6JA (GB)**

(74) Representative: **Rackham, Stephen Neil**
**GILL JENNINGS & EVERY,**
**Broadgate House,**
**7 Eldon Street**
**London EC2M 7LH (GB)**

(54) **Method and apparatus for measuring blood flow.**

(57) Blood flow, particularly cerebral blood flow, can be safely measured based on existence of a light absorption peak in the near infra-red (NIR) light region for normal water and the absence of such a peak for heavy water. Heavy water is intra-arterially injected into a patient. A patient's head (4) is irradiated with NIR light from a source (3). NIR light emerging from the patient's head (4) is detected by a photo multiplier tube (6) and changes in the light reception caused by changes in the concentration of normal water are monitored. A decrease in the amount of light absorption indicates passage of the heavy water through the area being measured. The time required for passage of the heavy water is used to calculate the blood flow.

FIG. 1

EP 0 615 723 A1

The present invention relates to a method and an apparatus for measuring cerebral blood flow and particularly cerebral blood flow (CBF) using heavy water as a tracer.

Proper supply of blood to the brain is essential for the brain, and consequently other body organs, to function properly and to maintain the life of the individual. Accurately measuring cerebral blood flow (CBF) is therefore an indispensable tool for diagnosing some critical patients. Several techniques have been proposed which use tracers for determining CBF in patients. In one method the tracer injected into a patient is a radioactive substance, commonly [133]xenon. A $\gamma$-ray sensor is attached to the patient's head for sensing changes in strength in radioactivity from the radioactive substance. A rapid increase and decrease in the radioactive strength would presume rapid blood flow. In this way, measured radioactivity values reveal CBF. In another technique the tracer injected into a patient is a light-absorbing pigment, such as cardio-green. Light is directed on the patient's head. Changes in the amount of light absorbed by the pigment are monitored to determine the rate at which the pigment flows, and indirectly the rate at which blood flows.

However there has been known a serious drawback with these techniques in that the radioactive and pigment tracers adversely affect living tissue. Countries and organizations often prohibit injecting these injurious tracers into human subjects. Even when circumstances allow their use, the potential for harm prevents repeated applications. Although measuring blood flow with tracers provides more accurate and stable measurements than the Doppler method, the above problem with using radioactive or pigment tracers severely limits their application in clinical situations.

In view of the foregoing, it is an object of the present invention to provide a method and apparatus for measuring a blood flow using a harmless substance as a tracer safely, accurately and conveniently to measure the blood flow.

To achieve the above and other objects, there is provided according to one aspect of the invention a method of measuring blood flow according to the present invention comprising the steps of (a) injecting into a first portion of a blood vessel a predetermined quantity of a liquid containing heavy water, (b) irradiating with light of a predetermined wavelength region a second portion downstream from the first portion, (c) detecting light emerging from the second portion, (e) converting the detected light into an electric signal, the electrical signal changing with changes in concentration of the heavy water at the second portion caused by injection of the liquid at the first portion, (f) detecting an amount of change of the electric signal, (g) obtaining a speed of movement of the liquid within the blood vessel based on the amount of change of the electrical signal, and (h) computing a blood flow based on the speed of movement of the liquid thus obtained.

Preferably, the method further comprises a step of obtaining a blood volume of a section of the blood vessel substantially between the first portion and the second portion, wherein the blood flow in the section is computed by dividing the blood volume by the speed of movement of the liquid.

According to another aspect of the invention, there is provided an apparatus for measuring a blood flow, comprising means for irradiating light of a predetermined wavelength region into a body organ; means for detecting light emerging from the body organ; means for converting the detected light into an electric signal, the electrical signal changing with changes in concentration of a heavy water injected into a blood vessel of the body organ; means for detecting an amount of change of the electric signal; means for obtaining a speed of movement of a liquid within the blood vessel based on the amount of change of the electrical signal; and means for computing a blood flow based on the speed of movement of the liquid thus obtained.

Preferably, the apparatus further comprises means for obtaining a blood volume in the body organ, wherein the blood flow in the body organ is computed by dividing the blood volume by the speed of movement of the liquid.

The above and other objects, features and advantages of the invention will become more apparent from reading the following description of the preferred embodiment taken in connection with the accompanying drawings in which:

Fig. 1 is a block diagram showing a cerebral blood flow (CBF) measuring device according to one embodiment of the present invention;

Fig. 2(a) is a graphical representation showing an absorption spectrum of water;

Fig. 2(b) is a graphical representation showing absorption spectra of both oxyhemoglobin and deoxyhemoglobin;

Fig. 3 is a graphical representation showing change in concentration of water resulting from injection of heavy water into a body organ;

Fig. 4 is a flow chart for illustrating operational sequences of the device shown in Fig. 1; and

Fig. 5 is a graphical representation showing second derivative curves of absorption spectra of both water and heavy water.

Referring to the accompanying drawings, a preferred embodiment of the invention will be described.

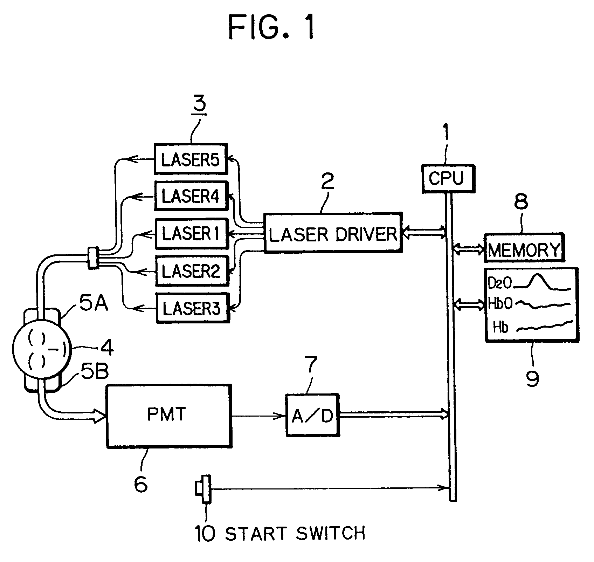

As can be seen in Fig. 1, a light source 3 is connected to a central processing unit (CPU) 1 via a laser driver 2. The light source 3 outputs first through fifth lasers in time sharing manner according to signals from the laser driver 2. The first through fifth lasers have $\lambda_1$ through $\lambda_5$ wavelengths, respectively, as listed below:

| first laser | $\lambda_1$ | 905 nm |
|---|---|---|
| second laser | $\lambda_2$ | 975 nm |
| third laser | $\lambda_3$ | 1040 nm |
| fourth laser | $\lambda_4$ | 775 nm |
| fifth laser | $\lambda_5$ | 825 nm |

The first through third lasers, with first through third wavelengths $\lambda_1$ to $\lambda_3$ respectively, serve to measure changes in the absorption peak of water. The fourth and fifth lasers, with the fourth and fifth wavelength $\lambda_4$ and $\lambda_5$ respectively, serve to measure changes in concentration of both oxyhemoglobin ($HbO_2$) and deoxyhemoglobin (Hb) to obtain cerebral blood volume (CBV). When measurement of CBV does not need to be carried out simultaneously, only the first to third lasers are outputted from the light source 3.

The light source 3 is optically connected to a first flexible optical fiber bundle attached at a surface of a patient's head 4 by an emit-side fastener 5A. Light outputted by the light source 3 is directed through the first flexible optical fiber bundle into the patient's head 4. A detect-side fastener 5B is attached to the patient's head 4 at a surface opposite to the emit-side fastener attachment position. The detect-side fastener 5b supports a second flexible optical fiber bundle which is optically connected to a photomultiplier tube 6. The detect-side fastener 5b collects light that emerges from the patient's head after being directed therein at the emit-side fastener 5a and directs the collected light to the photomultiplier tube 6 where it is converted into an electric signal.

The output of the photomuliplier tube 6 is electrically connected to an A/D converter 7 which in turn is connected to the CPU 1. The CPU 1 connects a memory 8, an output device 9 and a start switch 10. The electric signal from the photomultiplier tube 6 is subjected to analog-to-digital conversion in the A/D converter 7 and the resultant digital signal is stored in the memory 8 under the aegis of the CPU 1. The memory 8 stores various parameters needed for computing a cerebral blood flow (CBF) and programs for running the CPU 1. The start switch 10 is provided for notifying the CPU 1 of the start of measurement.

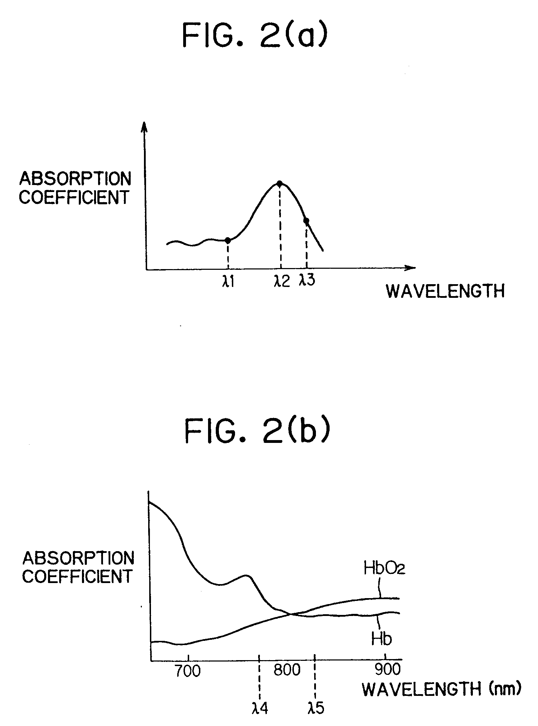

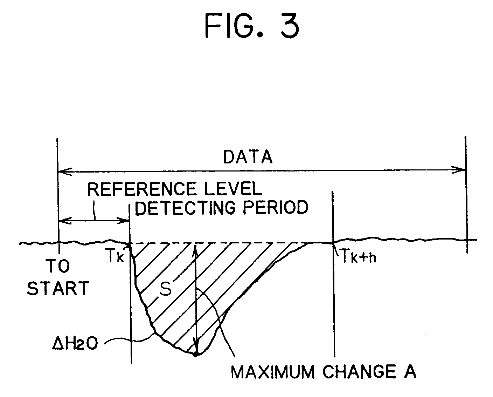

The CBF measurement performed according to the preferred embodiment will next be described while referring to Figs. 2 and 3.

The present invention uses heavy water as a tracer to determine CBF. Heavy water, also known as deuterium oxide ($D_2O$), is a compound of hydrogen and oxygen containing a higher proportion of the hydrogen isotope deuterium than does naturally occurring water. Certain amounts of heavy water can be injected into the bloodstream without adverse affects because heavy water affects living tissue in the same manner and to the same degree as does naturally occurring water. Note the main component of blood is water. In the preferred embodiment of the present invention, heavy water is injected into the bloodstream as a saline solution.

Although living tissue is relatively transparent to near-infrared (NIR) light, naturally occurring water starts absorbing light in the NIR range at around the first wavelength $\lambda_1$ (905 nm) as graphically shown in Fig. 2-(a). Absorption increases until a peak is reached near the second wavelength $\lambda_2$ (975 nm) whereafter absorption declines until the third wavelength $\lambda_3$ (1040 nm).

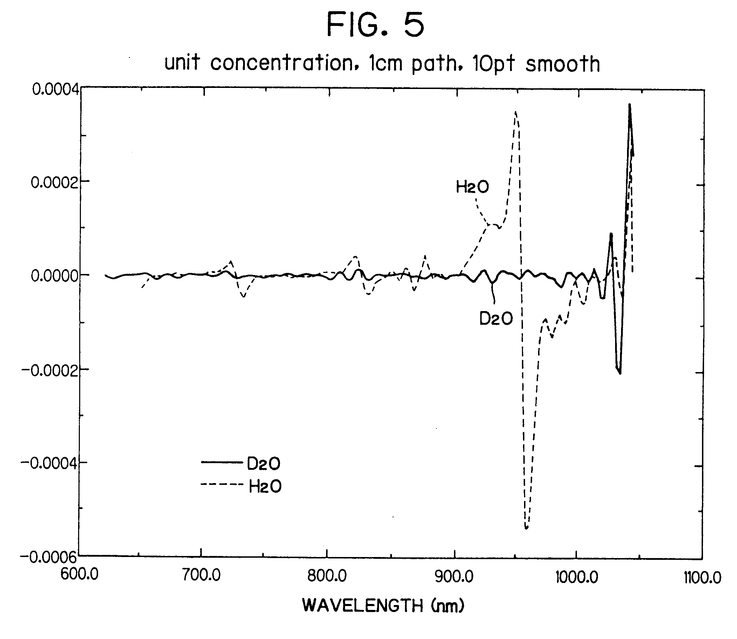

Heavy water does not substantially absorb light in this wavelength range. Fig. 5 shows the second derivative (second order differential) curves of light absorption spectra for naturally occurring water and heavy water at the NIR wavelength range. As shown in the figure, naturally occurring water shows a typical absorption peak at around 975 nm, whereas this peak is lacking in heavy water. Changes in concentration of naturally occurring water ($\Delta H_2O$) and heavy water ($\Delta D_2O$) caused by heavy water being interarterially injected into the bloodstream are in almost complementary relation in a short period of time. That is, any increase or decrease in the concentration of naturally occurring water translates to an equal but opposite change in concentration of heavy water. This relationship can be equationally described as $\Delta H_2O = -\Delta D_2O$.

Further, the heavy water intraarterially injected into the patient's bloodstream flows with the bloodstream flow, that is, does not substantially disperse evenly throughout the body within a limited period of time, and therefore remains concentrated within one area of the bloodstream flow. While the area of the bloodstream flow which includes the concentration of heavy water flows past the area of the head being measured, the amount of light absorbed near 975 nm will decrease because of the decrease in concentration of naturally occurring water. In this way, the duration of time $T_{tr}$ required for the area of the bloodstream flow containing the heavy water to pass through the area being measured can be theoretically determined.

The duration of time $T_{tr}$ can be obtained by directly monitoring the level of light absorption. It is, however, preferable that the passing time of the heavy water be calculated from the change in the level of the second derivative curve of the absorption spectra at 975 nm, because the light transilluminated through the patient's head contains spectral distortions caused by the components of the patient's head 4 other than blood, such as bone and other tissue. Such distortions are superimposed on the real information carrying light as a zero-th order or first order offset. To eliminate such offset and accurately monitor changes in light absorption caused only by heavy water, changes in the amplitude of the second derivative of absorption spectra at 975 nm are used for determining the passing time of the heavy water.

The second derivative (difference value) E of laser light at wavelength 975 nm can be expressed with the following equation:

$$E = \{OD(\lambda 2) - OD(\lambda 1)\} - \{OD(\lambda 3) - OD(\lambda 2)\}$$

$$= -OD(\lambda 1) + 2OD(\lambda 2) - OD(\lambda 3)$$

$$= L\{-\alpha(\lambda 1) + 2\alpha(\lambda 2) - \alpha(\lambda 3)\}\Delta H_2O$$

$$\therefore \ \Delta H_2O = \frac{\{-OD(\lambda 1) + 2OD(\lambda 2) - OD(\lambda 3)\}}{L\{-\alpha(\lambda 1) + 2\alpha(\lambda 2) - \alpha(\lambda 3)\}}$$

where $OD(\lambda_i)$ is an amount of light absorption at wavelength $\lambda$ (i = 1, 2, 3...), $\alpha(\lambda_i)$ is an absorption coefficient of naturally occurring water at wavelength $\lambda_i$, and $\Delta H_2O$ is a change in concentration of naturally occurring water. In the above equation, the difference between the amounts of light absorption at two discrete wavelengths, i.e., $\{OD(\lambda 2) - OD(\lambda 1)\}$ or $\{OD(\lambda 3) - OD(\lambda 2)\}$, reveals the first order differential amount of light absorption spectra, and the difference between the first order differential amounts, i.e., $\{OD(\lambda 2) - OD(\lambda 1)\} - \{OD(\lambda 3) - OD(\lambda 2)\}$, reveals the second order differential at the wavelength $\lambda_2$.

In order to implement computation of $\Delta H_2O$, the first through third lasers with wavelengths 905 nm ($\lambda_1$), 975 ($\lambda_2$), and 1040 nm ($\lambda_3$) respectively are sequentially (for example, at every 10 microseconds) transilluminated through the patient's head and the absorption amounts at the respective wavelengths of the light emerging from the head are measured, whereby $OD(\lambda_1)$, $OD(\lambda_2)$ and $OD(\lambda_3)$ are obtained. The data thus obtained are stored in the memory 8. CPU 1 substitutes the data into the equation above to obtain $\Delta H_2O$. The above measurements and computations are repeatedly carried out.

CBF can be determined by the following formula:

$$CBF = CBV/T_{tr}$$

where CBF is the cerebral blood flow and CBV is the cerebral blood volume.

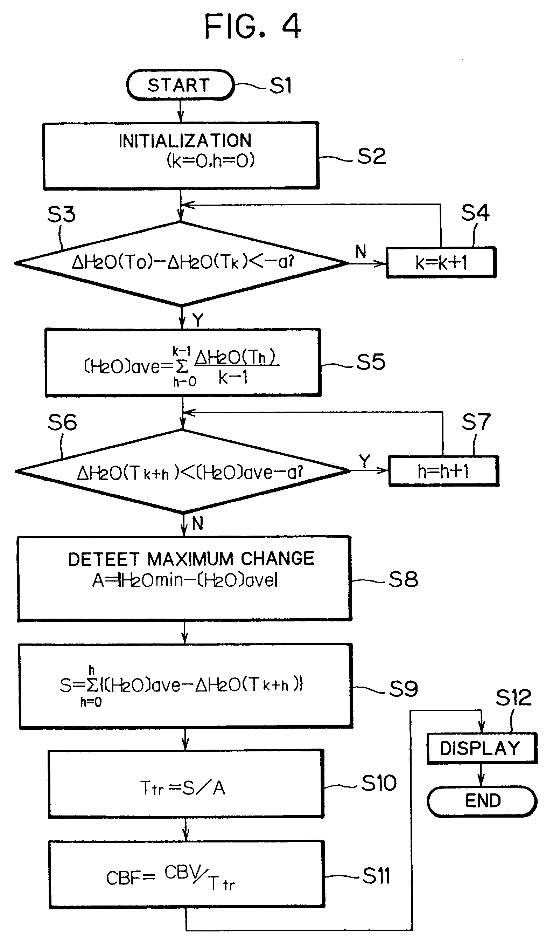

Next, the method of measuring CBF by the device shown in Fig. 1 will explained while referring to Figs. 3 and 4. First, an operator presses the start switch 10 to notify the CPU 1 of the start of measurement and computation in step 1. Next, a predetermined amount of saline solution containing heavy water is intraarterially injected into the patient. Then, initialization is performed in step 2 to zero parameters k and h. Next, the time $T_k$ when $\Delta H_2O$ starts decreasing caused by the injected saline solution containing heavy water is detected in steps 3 and 4. The time $T_k$ is determined as such a time instant when the concentration of naturally occurring water falls a predetermined value $\underline{a}$ below a reference value. The reference value used herein is the value of $\Delta H_2O$ at the time $T_0$ when the start switch 10 is pressed. The value $\underline{a}$ is set taking the amount of noise into consideration, but should generally be set to about 1/10 the maximum measured decrease.

When $T_k$ is detected, the average of the change in concentration of $H_2O$ between $T_0$ and $T_{k-1}$ is calculated and this average is set as a reference level $[H_2O]_{ave}$ in step 5. After $T_k$, change in $H_2O$ concentration will be less than $[H_2O]_{ave}$ as seen from Fig. 3. The time instant $T_{k+h}$ when the value of $\Delta H_2O$ becomes larger than $[H_2O]_{ave}$ minus $\underline{a}$ is detected in steps 6 and 7. At this time, monitoring of the change in $H_2O$ concentration is stopped and values required later are calculated. That is, A, the maximum change in $H_2O$ concentration from the reference level $[H_2O]_{ave}$, and S, an accumulation of the change in $H_2O$

concentration that occurs between $T_k$ and $T_{k+h}$, are computed in steps 8 and 9, respectively. $T_{tr}$, the duration of time from $T_k$ to $T_{k+h}$, that is, the time required for the injected heavy water to pass the area being measured, is determined by dividing S by A in step 10. The CBF is determined by dividing CBV, as determined previously by separate measuring methods, by $T_{tr}$ in step 11. The result is outputted by the output device 9 in step 12, whereupon the calculation algorithm is completed. The series of computations as described above are performed concurrently with the measurement of light absorption amount in the respective wavelengths.

Non-invasive quantitation of CBV is possible by using, for example, the NIR spectroscopy techniques described by J. S. Wyatt et al in their article "Quantitation of cerebral blood volume in human infants by near-infrared spectroscopy" on pages 1086 - 1091 in the 1990 Journal for the American Physiological Society. In view of the fact that absorption spectra of oxyhemoglobin ($HbO_2$) and deoxyhemoglobin (Hb) at wavelengths $\lambda_4$ (775 nm) and $\lambda_5$ (825 nm) are different as can be seen from Fig. 2(b), the light with wavelengths $\lambda_4$ and $\lambda_5$ are transilluminated into the patient's head from one side thereof and the light emerging from the head is detected at the other side. From the data thus detected, changes in concentration of the oxyhemoglobin and deoxyhemoglobin can be obtained. CBV can be obtained from the changes in concentration of the oxyhemoglobin and deoxyhemoglobin and an arterial oxygen saturation ($SaO_2$) obtained from a pulse oximeter well known in the art. By measuring the arterial oxygen saturation ($SaO_2$) simultaneously, the CBV can be computed with the device shown in Fig. 1.

Although the present invention has been described with reference to measurement of cerebral blood flow, the blood flow in various body organs can also be measured.

**Claims**

1. A method of measuring a blood flow, comprising the steps of:

injecting into a first portion of a blood vessel a pre-determined quantity of a liquid containing heavy water;

irradiating with light of a pre-determined wavelength region a second portion downstream from the first portion;

detecting light emerging from the second portion;

converting the detected light into an electrical signal, the electrical signal changing with changes in concentration of the heavy water at the second portion caused by injection of the liquid at the first portion;

monitoring the change of the electrical signal;

obtaining a speed of movement of the liquid within the blood vessel based on the monitored change of the electrical signal; and,

computing a blood flow based on the speed of movement of the liquid thus obtained.

2. A method according to claim 1, further comprising the step of obtaining the blood volume of a section substantially between the first portion and the second portion, and wherein the blood flow in the section is computed by dividing the blood volume by the speed of movement of the liquid.

3. A method of measuring a cerebral blood flow comprising the steps of:

injecting into a first portion of an artery a pre-determined quantity of a liquid containing heavy water;

irradiating with light of a pre-determined wavelength region a cerebral area downstream from the first portion;

detecting light emerging from the cerebral area;

converting the detected light into an electrical signal, the electrical signal changing with changes in concentration of the heavy water in the cerebral area caused by injection of the liquid at the first portion;

detecting a change of the electric signal;

obtaining the speed of movement of the liquid within the cerebral area from the detected change; and,

computing a cerebral blood flow based on the speed of movement of the liquid thus obtained.

4. A method according to claim 3, further comprising the step of obtaining the blood volume of the cerebral area, and wherein the blood flow in the cerebral area is computed by dividing the blood volume by the speed of movement of the liquid.

**5.** A method according to any one of the preceding claims, further comprising the step of displaying the computed blood flow.

**6.** An apparatus for measuring a blood flow, comprising:

means (2, 3, 5A) for irradiating light of a pre-determined wavelength region into a body organ (4);

means (5B, 6) for detecting light emerging from the body organ (4);

means for converting the detected light into an electrical signal, the electrical signal changing with changes in concentration of heavy water injected into a blood vessel leading to the body organ;

means for monitoring the change in the electrical signal;

means for obtaining a speed of movement of a liquid within the blood vessel based on the monitored change of the electrical signal; and,

means for computing a blood flow based on the speed of movement of the liquid thus obtained.

**7.** An apparatus according to claim 6, further comprising means (9) for displaying the computed blood flow.

**8.** A method or apparatus according to any one of the preceding claims, wherein the pre-determined wavelength region is chosen to be one in which naturally occurring water which is a major component of blood absorbs more light than does heavy water.

**9.** A method or apparatus according to any one of the preceding claims, wherein the pre-determined wavelength region is a near infra-red region.

**10.** A method or apparatus according to any one of the preceding claims, wherein the pre-determined wavelength region is centered on a wavelength of 975 nm.

# FIG. 1

# FIG. 2(a)

# FIG. 2(b)

# FIG. 3

DATA

REFERENCE LEVEL
DETECTING PERIOD

TO
START

Tk

S

ΔH2O

Tk+h

MAXIMUM CHANGE A

# FIG. 4

START — S1

INITIALIZATION
(k=0,h=0) — S2

S3

$\Delta H_2O(T_0)-\Delta H_2O(T_k)<-a?$ — S4 k=k+1 (N)

Y

$(H_2O)ave=\sum\limits_{h-0}^{k-1}\dfrac{\Delta H_2O(T_h)}{k-1}$ — S5

S6

$\Delta H_2O(T_{k+h})<(H_2O)ave-a?$ — S7 h=h+1 (Y)

N

DETEET MAXIMUM CHANGE
$A=|H_2Omin-(H_2O)ave|$ — S8

$S=\sum\limits_{h=0}^{h}\{(H_2O)ave-\Delta H_2O(T_{k+h})\}$ — S9

S12

DISPLAY

$T_{tr}=S/A$ — S10

END

$CBF=\dfrac{CBV}{T_{tr}}$ — S11

# FIG. 5

unit concentration, 1cm path, 10pt smooth

EP 0 615 723 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 502 270 (HAMAMATSU PHOTONICS K.K.) * page 4, column 5 - line 29; figures 1-5 * | 1,5-8 | A61B5/026 A61B5/0275 G01F1/712 |
| Y | BIOMEDIZINISCHE TECHNIK vol. 33, no. 1-2, February 1988, BERLIN DE pages 6 - 9 J.BÖCK ET AL. 'Simultaneous Fiberoptic Detection of the Kinetics of Heavy Water and Indocyanine Green Dye in Arterial Vessels' * the whole document * | 1,5-8 | |
| A | US-A-3 677 648 (J.DORSCH) * the whole document * | 1,5-8 | |
| A | EP-A-0 290 274 (HAMAMATSU PHOTONICS K.K.) * the whole document * | 1,3,6,9 | |
| A | Database INSPEC, IEE, LONDON, G.B. INSPEC No.1595229 M.Apfelbaum et al : "Infrared spectrometry measurement of slight heavy water enrichment in natural water" * abstract * | 1,3,6 | TECHNICAL FIELDS SEARCHED (Int. Cl.5) A61B G01F |
| A | & JOURNAL DE BIOPHYSIQUE & MEDECINE NUCLEAIRE vol. 4, no. 2, 1980, (FR) pages 91 - 94 M. APFELBAUM ET AL. 'Infrared spectrometry measurement of slight heavy water enrichment in natural water' | 1,3,6 | |

The present search report has been drawn up for all claims

| Place of search | Date of completion of the search | Examiner |
|---|---|---|
| THE HAGUE | 26 JULY 1993 | HUNT B.W. |

EPO FORM 1503 03.82 (P0401)